# FASCICULE DE BREVET EUROPEEN

(11) **EP 0 803 489 B1**
(45) Date de publication et mention de la délivrance du brevet: **30.08.2000**
(21) Numéro de dépôt: 97400919.3
(22) Date de dépôt: 23.04.1997
(51) Int. Cl.: C07C 2/30

(54) **Procédé amélioré de conversion de l'éthylène en oléfines alpha légères avec utilisation d'additifs a base de sels d'ammonium quaternaire**
Verbessertes Verfahren zur Umwandlung von Ethylen in niedrigen Alpha-Olefinen unter Verwendung von Additiven die auf quaternären Ammoniumsalzen basieren
Improved process for the conversion of ethylen to light aplha-olefins using additives which are based on quaternary salts

(30) Priorité: 26.04.1996 FR 9605399
(43) Date de publication de la demande: 29.10.1997
(73) Titulaire: INSTITUT FRANCAIS DU PETROLE, 92852 Rueil-Malmaison Cedex (FR)
(72) Inventeur: Commereuc, Dominique, 92190 Meudon (FR); Panzarella, Pierre, 38240 Maylan (FR); Glaize, Yves, 69360 Saint Symphorien D'Ozon (FR); Saussine, Lucien, 78290 Croissy Sur Seine (FR); Hugues, François, 69390 Vernaison (FR)

(56) Documents cités:
- EP-A- 0 578 541
- US-A- 4 855 525
- US-A- 5 292 979

## Description

L'objet de la présente invention est un procédé amélioré de production d'oléfines alpha légères par oligomérisation de l'éthylène, grâce à l'utilisation d'additifs à base de sels d'ammonium quaternaire.

Dans les procédés d'oligomérisation de l'éthylène en oléfines alpha légères au moyen de catalyseurs solubles à base de zirconium, il se forme de petites quantités de polymère solide qui se déposent et collent sur la surface du réacteur et des tubes d'échangeurs de chaleur et qui sont très nuisibles à la bonne marche du procédé car elles réduisent les transferts de chaleur et nécessitent l'arrêt fréquent du réacteur afin de les éliminer.

Un moyen connu pour réduire la quantité de polymère formé consiste à injecter de faibles quantités d'hydrogène (US 4 855 525).

Il a maintenant été trouvé que si la réaction d'oligomérisation de l'éthylène est conduite en présence d'additifs composés par des sels d'ammonium quaternaire, la quantité de polymère solide sous-produit est réduite et surtout son adhérence aux parois du réacteur et des échangeurs est considérablement diminuée.

L'invention a ainsi pour objet un procédé amélioré de conversion de l'éthylène en oléfines alpha légères, dans lequel, dans une enceinte réactionnelle, on met l'éthylène en contact avec une solution d'un catalyseur obtenu par mélange d'un composé de zirconium avec un composé organique choisi dans la classe des acétals et des cétals, des esters, des cétones, des éthers, des amines, des nitriles, des anhydrides, des chlorures d'acides, des amides, des aldéhydes, des thioéthers, des sulfures et des disulfures d'alkyle, des thiophènes, des thiourées, des phosphines, et avec un composé chloré ou bromé d'aluminium hydrocarbyl, et en présence d'au moins un additif choisi dans le groupe formé par les sels d'ammonium quaternaire.

De préférence, on met l'éthylène en contact avec une solution d'un catalyseur obtenu par mélange d'au moins un composé de zirconium avec au moins un composé organique choisi dans la classe des acétals et des cétals et avec au moins un composé chloré ou bromé d'aluminium hydrocarbyl, et en présence d'au moins un additif choisi dans le groupe formé par les sels d'ammonium quaternaire.

Les éléments de la solution de catalyseur sont décrits par exemple dans la demande de brevet EP 578 541 dont les enseignements sont ci-inclus. Plus précisément, un catalyseur préféré est obtenu par mélange :
- d'un composé de zirconium de formule ZrXₓY_{y}O_{z} dans lequel X est un atome de chore ou de brome, Y est un radical choisi dans le groupe formé par les alkoxy RO⁻, les amido R₂N⁻, les carboxylates RCOO-, où R est un radical hydrocarbyl comprenant de 1 à 30 atomes de carbone, x et y peuvent prendre les valeurs entières de 0 à 4 et z est égal à 0 ou 0,5, la somme x + y + 2z étant égale à 4,
- avec un composé organique de formule (R₁')(R₂')C(OR₁)(OR₂) dans laquelle R₁' et R₂' sont constitués par un atome d'hydrogène ou un radical hydrocarbyl comprenant de 1 à 30 atomes de carbone, R₁ et R₂ sont des radicaux hydrocarbyl comprenant de 1 à 30 atomes de carbone,
- et avec un composé d'aluminium de formule AIR'ₙX₃₋ₙ dans laquelle R" est un radical hydrocarbyl comprenant de 1 à 6 atomes de carbone, X est un atome de chlore ou de brome, et n est un nombre compris entre 1 et 2.

Les composants du catalyseur peuvent être mis en contact dans un ordre quelconque dans un solvant choisi dans le groupe formé par les hydrocarbures aliphatiques tels que l'hexane ou l'heptane, les hydrocarbures aromatiques tels que le toluène ou l'ortho-xylène, et les sous-produits d'oligomérisation tels que les oligomères supérieurs. De préférence, le composé de zirconium est d'abord mélangé avec l'acétal ou le cétal, puis le composé d'aluminium est ajouté au mélange.

De façon particulièrement avantageuse, la solution de catalyseur résulte de l'interaction d'un mélange d'au moins un composé de zirconium, tel que par exemple le tétrachlorure de zirconium, et d'au moins un composé organique choisi dans la classe des acétals et des cétals, résultant de la condensation d'un aldéhyde ou d'une cétone avec un monoalcool ou un polyalcool, tel que par exemple le di-(éthyl-2-hexyloxy)-2,2-propane, avec au moins un composé chloré ou bromé d'aluminium hydrocarbyl, tel que par exemple le sesquichlorure d'éthylaluminium.

Le rapport molaire entre l'acétal ou le cétal et le composé de zirconium est de 0,1:1 à 5:1, et de préférence de 0,5:1 à 2:1. Le rapport molaire entre le composé d'aluminium et le composé de zirconium est de 1:1 à 100:1, de préférence de 5:1 à 50 :1. La concentration de zirconium dans la solution catalytique ainsi préparée est avantageusement comprise entre 10⁻⁴ et 0,5 mole par litre, et de préférence entre 2. 10⁻³ et 0,1 mole par litre. La température à laquelle les trois composants sont mélangés est normalement comprise entre -10 et +150 °C, de préférence entre 0 et +80°C, par exemple à une température voisine de l'ambiante (15 à 30 °C). Le mélange peut être effectué sous une atmosphère d'éthylène ou de gaz inerte.

Une autre solution catalytique, telle que décrite dans le brevet US 5 292 979, convient également. Elle résulte du mélange d'un alkylzirconate et d'un éther avec un rapport molaire éther / zirconate de 0,5 à 10, avec un halogénure d'hydrocarbylaluminium.

On peut également citer les solutions catalytiques décrites dans le brevet US 4 855 525, résultant de l'interaction d'un alkylaluminium avec le mélange d'un tétrahalogénure (chlorure, bromure) de zirconium et d'un composé organique choisi dans le groupe formé par les esters, cétones, éthers, amines, nitriles, anhydrides, chlorures d'acide, amides et aldéhydes.

Les compositions catalytiques décrites dans la demande de brevet EP 241.596 sont également utilisables. Ce sont des mélanges d'halogénures de zirconium avec un composé organique de l'aluminium et d'une base de Lewis choisie dans le groupe formé par les thioéthers, les disulfures d'alkyle, les thiophènes, les thiourées, les sulfures, les phosphines et les amines primaires.

Toutes les compositions citées ci-dessus le sont à titre illustratif et non limitatif.

La solution catalytique obtenue peut être utilisée telle quelle, ou bien peut être diluée par exemple par addition des produits de la réaction d'oligomérisation.

Les sels d'ammonium quaternaire utilisés selon l'invention répondent à la formule générale [(R₁R₂R₃R₄)N⁺]X⁻ dans laquelle R₁, R₂, R₃ et R₄ sont des radicaux hydrocarbyle, identiques ou différents, par exemple des radicaux alkyle, cycloalkyle, aryle, cycloalkyle ou aryle substitués par un groupe alkyle, comprenant de 1 à 30 atomes de carbone, et X est un anion monovalent, par exemple un halogénure ou un hydroxyde. On peut citer à titre d'exemple le chlorure de tétraéthylammonium, le bromure de tétraéthylammonium, le chlorure de triméthyl-cétylammonium, le bromure de triméthyl-cétylammonium, le chlorure de diméthyl-dilaurylammonium, le chorure de méthyltrioctylammonium, le chlorure de méthyl-tridécylammonium, le chlorure de benzyl-diméthyl-cétylammonium. Les chlorures sont les sels préférés. Les sels d'ammonium quaternaire peuvent être mis en oeuvre tels quels (purs) ou sous forme d'une solution dans un milieu hydrocarboné choisi dans le groupe formé par les hydrocarbures et / ou par les oléfines alpha légères produit d'oligomérisation, et / ou par les sous-produits de la réaction tels que les oligomères supérieurs.

Que ce soit un procédé continu ou discontinu, les sels d'ammonium quaternaire, purs ou en solution, peuvent être introduits avant de procéder à la réaction d'oligomérisation de l'éthylène, par exemple ils peuvent être utilisés pour effectuer un traitement de passivation des parois de l'enceinte réactionnelle préalablement au démarrage de la réaction. Les parois de l'enceinte sont métalliques (métaux, aciers, alliages,...) et peuvent avoir subi des traitements de protection (polissage, vitrification,...) ou peuvent avoir été soumises à une protection anodique.

La passivation est effectuée selon toutes les techniques connues. Avantageusement, on charge l'enceinte avec une solution de 20 ppm à 5 % en poids d'additif dans un milieu hydrocarboné, le contact est maintenu de préférence sous agitation pendant 10 min à 10 h, de préférence 30 min à 3 h, à une température inférieure à la température d'ébullition du solvant, 20 à 100° C généralement et 30 à 80° C de préférence. La solution de passivation est ensuite généralement évacuée.

Les sels d'ammonium quaternaire, purs ou en solution, peuvent aussi être introduits de façon continue ou en discontinu pendant le déroulement de la réaction, par exemple en mélange avec la solution du zirconium, de préférence sous forme d'un flux indépendant des flux de catalyseur. Il peut être avantageux de combiner un traitement de passivation préalable de l'enceinte réactionnelle suivi d'une injection en continu ou en discontinu pendant le déroulement de la réaction.

La quantité de sels d'ammonium quaternaire mise en oeuvre pendant la réaction d'oligomérisation peut représenter de 1 partie par million en poids (ppm) à 5 % en poids, avantageusement de 1 ppm à 1 % et de préférence de 20 ppm à 5000 ppm, par rapport aux oligomères produits, que cette quantité soit introduite pendant la réaction (procédé continu) ou dans l'enceinte avant réaction (procédé discontinu).

La réaction d'oligomérisation de l'éthylène peut être mise en oeuvre à une température de 20 à 180 °C, de préférence de 40 à 150 °C et de manière encore plus préférée de 40 à 130 °C. La pression est de 0,5 à 15 MPa et de préférence de 1 à 10 MPa.

Dans un mode de mise en oeuvre de la réaction catalytique d'oligomérisation en discontinu, on introduit dans un réacteur (enceinte réactionnelle) muni des habituels systèmes d'agitation et de refroidissement l'additif avec la solution de catalyseur, par exemple un volume choisi de solution catalytique, préparée comme décrit ci-dessus, et, indépendamment, un volume choisi d'une solution de sel d'ammonium quaternaire, après quoi on pressurise par de l'éthylène et on ajuste la température à la valeur souhaitée. On alimente le réacteur par de l'éthylène à pression constante jusqu'à ce que le volume total de liquide produit remplisse presque complètement le réacteur. On détruit, après réaction, le catalyseur, par exemple par injection d'une amine, et on soutire et sépare les produits de la réaction et les solvants éventuels.

En cas d'opération en continu, on peut avantageusement commencer chaque marche par une passivation des parois du réacteur par un volume choisi d'une solution de sel d'ammonium quaternaire. Après avoir soutiré cette solution et avantageusement rincé le réacteur avec un hydrocarbure, la solution catalytique est injectée en continu en même temps, et de préférence indépendamment, qu'une solution de sel d'ammonium quaternaire et en même temps que l'éthylène. La température et la pression sont maintenues constantes au moyen de tout système de régulation habituel. L'effluent du réacteur est envoyé, après avoir été mis au contact avec une amine, dans une colonne de flash où l'effluent traité par l'amine est vaporisé, soit par élévation de température, soit par abaissement de la pression, soit par action simultanée sur la température et la pression, de façon à recueillir les alpha-oléfines dans la fraction vaporisée.

Il est souhaitable d'avoir un taux de vaporisation maximum, par exemple au moins 90 % du volume de l'effluent traité à l'amine est vaporisé, et de préférence 95 % ou plus, de manière à limiter la quantité de rejets qui devront être traités conformément aux impératifs d'environnement. Cependant ceci ne doit pas conduire à une température de vaporisation prohibitive, voire nuisible à la stabilité thermique des oléfines. On préfère respecter une température de vaporisation inférieure ou égale à 250 °C, et de préférence 200 °C.

Les produits lourds résultant de la vaporisation, et contenant le catalyseur désactivé, peuvent être incinérés, ou traités de toute manière conforme aux normes de l'environnement.

Les oligomères vaporisés sont selon les besoins dirigés vers un système de colonnes à distiller qui permet de séparer, d'une part l'éthylène non converti des oligomères, l'éthylène pouvant être renvoyé au réacteur d'oligomérisation, puis d'autre part les oligomères entre eux.

Les exemples suivants illustrent l'invention sans en limiter la portée.

### EXEMPLE 1

La réaction d'oligomérisation de l'éthylène est réalisée dans une unité pilote fonctionnant en continu, comprenant un réacteur d'un volume total de 1 litre, opérant avec un contrôle de niveau à 0,7 litre liquide, et équipé d'une agitation mécanique à entraînement pneumatique. Avant le démarrage de l'essai, on procède à une opération de passivation des parois du réacteur. Pour ce faire, on injecte dans le réacteur une solution de 0,2 g de chlorure de tri-(octyl/décyl)-méthylammonium (commercialisé sous le nom d'Adogen 464) dans 1 kg d'ortho-xylène, que l'on agite pendant 2 heures à une température de 90° C. A l'issue de ce laps de temps, on soutire la solution du sel d'ammonium.

La réaction d'oligomérisation de l'éthylène est ensuite conduite dans les conditions suivantes. Dans le réacteur, dont la température est régulée à 90° C au moyen d'une circulation d'huile, et dont la pression est maintenue à 7,5 MPa grâce à une vanne de détente située sur la ligne de sortie, on injecte en continu 25,1 g/h d'une solution de 2,47 g de chlorure de zirconium sublimé et de 3,18 g de di-(éthyl-2-hexyloxy)-2,2-propane dans 1,13 litre d'orthoxylène séché et désaéré, et 24,3 g/h d'une solution de 15,22 g de sesquichlorure d'éthylaluminium dans 1,12 litre d'ortho-xylène séché et désaéré. On injecte également en continu 300 g/h d'une solution de 0,0079 g de chlorure de tri-(octyl/décyl)-méthylammonium dans 1,14 litre d'ortho-xylène. Dans ces conditions, le débit d'éthylène à l'entrée du réacteur, asservi au contrôle de niveau, s'établit à 300 g/h. A la sortie du réacteur, on injecte en ligne, en continu, 25,5 g/h d'une solution de 47,6 g de laurylamine dans 1,14 litre d'ortho-xylène séché et désaéré. L'effluent traverse ensuite une colonne de flash fonctionnant à une température de 150 °C sous une pression de 0,3 MPa. La fraction flashée est envoyée à une colonne de stabilisation, et la fraction non flashée est collectée dans un pot de recette. Cette fraction non flashée, qui contient les résidus de catalyseur, une partie d'oligomères lourds constitués par des cires et un peu de polymère, représente un débit de 20 g/h.

Après 80 heures de marche, l'essai est arrêté intentionnellement. On arrête d'abord l'injection du catalyseur, ce qui a pour conséquence l'arrêt progressif de l'entrée d'éthylène, puis après 4 heures, on arrête l'injection de solvant contenant le sel d'ammonium et on laisse refroidir le réacteur. Après ouverture, on recueille 20 g de polymère, sous forme d'une couche granuleuse tapissant l'ensemble des internes du réacteur et très facile à détacher à la main. Ceci correspond à l'accumulation de en moyenne 0,25 g/h de polymère dans le réacteur.

### EXEMPLE 2 (Comparatif)

On réalise la réaction d'oligomérisation de l'éthylène en utilisant le même appareillage et le même mode opératoire que dans l'exemple 1, dans les mêmes conditions de température et de pression et en injectant dans le réacteur la même quantité de catalyseur. Cependant, on n'effectue pas de traitement préalable de passivation du réacteur et on n'injecte pas de sel d'ammonium en continu, c'est-à-dire qu'en sus du catalyseur, on injecte 300 g/h d'ortho-xylène pur. Le débit d'éthylène à l'entrée du réacteur se stabilise à 300 g/h. A la sortie du réacteur, l'effluent est traité comme dans l'exemple 1. La fraction non flashée, qui contient les résidus de catalyseur, une partie des oligomères lourds constitués par des cires et un peu de polymère, représente un débit de 18,5 g/h. L'essai a du être arrêté après 14 heures de marche seulement, par suite du blocage de l'agitation mécanique par le polymère formé. Après ouverture du réacteur, on recueille 8 g de polymère, sous forme d'une masse compacte dure et fortement adhérente, englobant une bonne partie des équipements intérieurs. Ceci correspond à l'accumulation de en moyenne 0,57 g/h de polymère dans le réacteur.

Par comparaison avec l'exemple 1, cet exemple de l'art antérieur montre que l'accumulation du polymère dans le réacteur est plus importante et que son adhérence est bien plus grande, ce qui provoque rapidement des perturbations nuisibles au bon fonctionnement du réacteur.

### EXEMPLE 3

La réaction d'oligomérisation de l'éthylène est réalisée dans le même appareillage que dans l'exemple 1, le réacteur ayant été passivé au préalable comme décrit dans ce même exemple.

Les conditions opératoires sont les suivantes. Dans le réacteur, dont la température est régulée à 90 °C au moyen d'une circulation d'huile, et dont la pression est maintenue à 7,5 MPa grâce à une vanne de détente située sur la ligne de sortie, on injecte en continu 18,1 g/h d'une solution de 7,74 g de chlorure de zirconium sublimé et de 9,96 g de di-(éthyl-2-hexyloxy)-2,2-propane dans 1,12 litre d'ortho-xylène séché et désaéré, et 35,5 g/h d'une solution de 31,81 g de sesquichlorure d'éthylaluminium dans 1,1 litre d'ortho-xylène séché et désaéré. On injecte également en continu 200 g/h d'une solution de 0,067 g de chlorure de tri-(octyl/décyl)-méthylammonium dans 1 kg d'un mélange 1/1 en volume d'heptane et d'ortho-xylène. Dans ces conditions, le débit d'éthylène à l'entrée du réacteur, asservi au contrôle de niveau, s'établit à 320 g/h. A la sortie du réacteur, on injecte en ligne, en continu, 39,5 g/h d'une solution de 84,23 g de laurylamine dans 1,04 litre d'ortho-xylène séché et désaéré. L'effluent traverse ensuite une colonne de flash fonctionnant à une température de 150 °C sous une pression de 0,3 MPa. La fraction flashée est envoyée à une colonne de stabilisation, et la fraction non flashée est collectée dans un pot de recette. Cette fraction non flashée, qui contient les résidus de catalyseur, une partie d'oligomères lourds constitués par des cires et un peu de polymère, représente un débit de 17,5 g/h. Après 68 heures de marche, l'essai est arrêté intentionnellement. On arrête d'abord l'injection du catalyseur, ce qui a pour conséquence l'arrêt progressif de l'entrée d'éthylène, puis après 4 heures, on arrête l'injection de solvant contenant le sel d'ammonium et on laisse refroidir le réacteur. Après ouverture, on recueille environ 1 g de polymère, sous forme d'une couche très fine tapissant l'ensemble des internes du réacteur et très facile à détacher avec le doigt. Ceci correspond à l'accumulation de en moyenne 0,015 g/h de polymère dans le réacteur.

## Revendications

1. Procédé de conversion de l'éthylène en oléfines alpha légères, dans lequel, dans une enceinte réactionnelle, on met l'éthylène en contact avec une solution d'un catalyseur obtenu par mélange d'un composé de zirconium avec un composé organique choisi dans la classe des acétals et des cétals, des esters, des cétones, des éthers, des amines, des nitriles, des anhydrides, des chlorures d'acides, des amides, des aldéhydes, des thioéthers, des sulfures et des disulfures d'alkyle, des thiophènes, des thiourées, des phosphines, et avec un composé chloré ou bromé d'aluminium hydrocarbyl, procédé caractérisé en ce qu'il se déroule en présence d'au moins un additif choisi dans le groupe formé par les sels d'ammonium quaternaire.

2. Procédé selon la revendication 1, caractérisé en ce que la solution de catalyseur résulte de l'interaction d'au moins un composé chloré ou bromé d'aluminium hydrocarbyl, avec un mélange d'au moins un composé de zirconium et d'au moins un composé organique choisi dans la classe des acétals et des cétals, résultant de la condensation d'un aldéhyde ou d'une cétone avec un monoalcool ou un polyalcool.

3. Procédé selon la revendication 2, caractérisé en ce que la concentration de zirconium dans la solution catalytique est comprise entre 10⁻⁴ et 0,5 mole par litre.

4. Procédé selon l'une des revendications 2 ou 3, caractérisé en ce que le rapport molaire entre le composé de l'aluminium et le composé du zirconium est compris entre 1:1 et 100:1.

5. Procédé selon l'une des revendications 2 à 4, caractérisé en ce que le rapport molaire entre l'acétal ou le cétal et le composé du zirconium est compris entre 0,1:1 et 5:1.

6. Procédé selon l'une des revendications précédentes, caractérisé en ce que le composé de zirconium est le tétrachlorure de zirconium.

7. Procédé selon l'une des revendications 2 à 6, caractérisé en ce que le cétal est le di-(éthyl-2-hexyloxy)-2,2-propane.

8. Procédé selon l'une des revendications précédentes, caractérisé en ce que le composé d'aluminium est le sesquichlorure d'éthylaluminium.

9. Procédé selon l'une des revendications précédentes, caractérisé en ce que le sel d'ammonium quaternaire est choisi dans le groupe formé par les halogénures ou les hydroxydes d'ammonium quaternaire.

10. Procédé selon l'une des revendications précédentes, caractérisé en ce que l'additif est choisi dans le groupe formé par les chlorures d'ammonium quaternaire.

11. Procédé selon l'une des revendications précédentes, caractérisé en ce que l'ammonium quaternaire comporte des radicaux hydrocarbyle, identiques ou différents, par exemple des radicaux alkyle, cycloalkyle, aryle, cycloalkyle ou aryle substitués par un groupe alkyle, comprenant de 1 à 30 atomes de carbone.

12. Procédé selon l'une des revendications précédentes, caractérisé en ce que l'additif est mis en oeuvre sous forme d'une solution dans un milieu hydrocarboné choisi dans le groupe formé par les hydrocarbures, les oléfines alpha légères, les oligomères supérieurs.

13. Procédé selon l'une des revendications 1 à 11, caractérisé en ce que l'additif est mis en oeuvre à l'état pur.

14. Procédé selon l'une des revendications précédentes, caractérisé en ce que, avant de procéder à la conversion, l'additif est introduit dans l'enceinte réactionnelle pour effectuer un traitement de passivation des parois de l'enceinte.

15. Procédé selon l'une des revendications précédentes, caractérisé en ce que l'additif est introduit pendant le déroulement de la réaction de conversion, le procédé étant continu.

16. Procédé selon l'une des revendications 1 à 14, caractérisé en ce que, le procédé étant discontinu, l'additif est introduit dans l'enceinte réactionnelle avec la solution de catalyseur.

17. Procédé selon l'une des revendications précédentes, caractérisé en ce que l'additif est introduit indépendamment de la solution de catalyseur.

18. Procédé selon l'une des revendications précédentes, caractérisé en ce que la quantité de sel d'ammonium quaternaire mise en oeuvre pendant la réaction de conversion est de 1 ppm à 5 % en poids par rapport aux oligomères produits.

19. Procédé selon l'une des revendications précédentes, caractérisé en ce que la quantité de sel d'ammonium quaternaire mise en oeuvre pendant la réaction de conversion est de 20 ppm à 5000 ppm par rapport aux oligomères produits.

20. Procédé selon l'une des revendications précédentes, caractérisé en ce que le procédé se déroule à une température comprise entre 20 et 180° C sous une pression de 0,5 à 15 MPa.

## Patentansprüche

1. Verfahren zur Umwandlung von Ethylen in α-Niederolefine, wobei man in einer Reaktionszelle das Ethylen in Kontakt mit einer Lösung eines Katalysators bringt, der durch Mischen einer Zirkoniumverbindung mit einer organischen Verbindung, ausgewählt aus der Gruppe der Acetale und der Ketale, der Ester, der Ketone, der Ether, der Amine, der Nitrile, der Anhydride, der Säurechloride, Amide, Aldehyde, Thioether, Alkylsulfide und -disulfide, Thiophene, Thioharnstoffe, Phosphine, und mit einer chlorierten oder bromierten Aluminium-Kohlenwasserstoffverbindung erhalten ist, wobei das Verfahren dadurch gekennzeichnet ist, daß es in der Anwesenheit von wenigstens einem Additiv erfolgt, das ausgewählt ist aus der Gruppe, gebildet von den quartären Salzen des Ammoniums.

2. Verfahren nach Anspruch 1, dadurch gekennzeichnet, daß die Katalysatorlösung aus der Wechselwirkung von wenigstens einer chlorierten oder bromierten Aluminium-Kohlenwasserstoffverbindung mit einer Mischung von wenigstens einer Zirkoniumverbindung und wenigstens einer organischen Verbindung resultiert, die ausgewählt aus der Gruppe der Acetale und Ketale, die aus der Kondensation eines Aldehyds oder eines Ketons mit einem einwertigen Alkohol oder einem mehrwertigen Alkohol resultiert.

3. Verfahren nach Anspruch 2, dadurch gekennzeichnet, daß die Konzentration an Zirkonium in der katalytischen Lösung in einem Bereich von 10⁻⁴ bis 0,5 Mol/l liegt.

4. Verfahren nach einem der Ansprüche 2 oder 3, dadurch gekennzeichnet, daß das molare Verhältnis zwischen der Aluminiumverbindung und der Zirkoniumverbindung zwischen 1:1 und 100:1 liegt.

5. Verfahren nach einem der Ansprüche 2 bis 4, dadurch gekennzeichnet, daß das molare Verhältnis zwischen dem Acetal oder dem Ketal und der Zirkoniumverbindung zwischen 0,1:1 und 5:1 liegt.

6. Verfahren nach einem der vorhergehenden Ansprüche, dadurch gekennzeichnet, daß es sich bei der Zirkoniumverbindung um das Zirkoniumtetrachlorid handelt.

7. Verfahren nach einem der Ansprüche 2 bis 6, dadurch gekennzeichnet, daß es sich bei dem Ketal um Di(ethyl-2-hexyloxy)-2,2-propan handelt.

8. Verfahren nach einem der vorhergehenden Ansprüche, dadurch gekennzeichnet, daß es sich bei der Aluminiumverbindung um das Ethylaluminium-Sesquichlorid handelt.

9. Verfahren nach einem der vorhergehenden Ansprüche, dadurch gekennzeichnet, daß das quartäre Ammoniumsalz ausgewählt ist aus der Gruppe, bestehend aus den Halogeniden oder den Hydroxiden quartären Ammoniums.

10. Verfahren nach einem der vorhergehenden Ansprüche, dadurch gekennzeichnet, daß das Additiv ausgewählt ist aus der Gruppe, bestehend aus den Chloriden quartären Ammoniums.

11. Verfahren nach einem der vorhergehenden Ansprüche, dadurch gekennzeichnet, daß das quartäre Ammonium radikalische Kohlenwasserstoffe beinhaltet, identisch oder verschieden, beispielsweise Alkyl-, Cycloalkyl-, Aryl-, mit einer Alkylgruppe mit 1 bis 30 Kohlenstoffatomen substituierte Cycloalkyl- oder Arylradikale.

12. Verfahren nach einem der vorhergehenden Ansprüche, dadurch gekennzeichnet, daß das Additiv in Form einer Lösung in einem Kohlenwasserstoffmedium bereitgestellt ist, ausgewählt aus der Gruppe, bestehend aus den Kohlenwasserstoffen, α-Niederolefinen, höheren Oligomeren.

13. Verfahren nach einem der Ansprüche 1 bis 11, dadurch gekennzeichnet, daß das Additiv in reinem Zustand bereitgestellt ist.

14. Verfahren nach einem der vorhergehenden Ansprüche, dadurch gekennzeichnet, daß vor dem Beginn der Umwandlung das Additiv in die Reaktionszelle eingeführt wird, um eine Passivierungsbehandlung der Wände der Zelle durchzuführen.

15. Verfahren nach einem der vorhergehenden Ansprüche, dadurch gekennzeichnet, daß das Additiv während der Durchführung der Umwandlungsreaktion zugegeben wird, wobei das Verfahren weitergeführt wird.

16. Verfahren nach einem der Ansprüche 1 bis 14, dadurch gekennzeichnet, daß das Verfahren unterbrochen wird, das Additiv in die Reaktionszelle mit der Katalysatorlösung eingegeben wird.

17. Verfahren nach einem der vorhergehenden Ansprüche, dadurch gekennzeichnet, daß das Additiv unabhängig von der Katalysatorlösung zugegeben wird.

18. Verfahren nach einem der vorhergehenden Ansprüche, dadurch gekennzeichnet, daß die Menge an quartärem Ammoniumsalz, das während der Umwandlungsreaktion bereitgestellt wird, im Bereich von 1 ppm bis 5 Gew.-% liegt, bezogen auf die oligomeren Produkte.

19. Verfahren nach einem der vorhergehenden Ansprüche, dadurch gekennzeichnet, daß die Menge an quartärem Ammoniumsalz, das während der Umwandlungsreaktion bereitgestellt wird, im Bereich von 20 ppm bis 5000 ppm liegt, bezogen auf die oligomeren Produkte.

20. Verfahren nach einem der vorhergehenden Ansprüche, dadurch gekennzeichnet, daß das Verfahren bei einer Temperatur stattfindet, die im Bereich zwischen 20 und 180°C bei einem Druck von 0,5 bis 15 MPa erfolgt.

## Claims

1. A process for the conversion of ethylene into light alpha olefins wherein, in a reaction enclosure, the ethylene is brought into contact with a solution of a catalyst obtained by mixing a compound of zirconium with an organic compound selected from the class of acetals and cetals, esters, ketones, ethers, amines, nitriles, anhydrides, chlorides of acids, amides, aldehydes, thioethers, alkyl sulphides and disulphides, thiophenes, thioureas, and phosphines, and with a chloro or bromo compound of aluminium hydrocarbyl, the process being characterised in that it takes place in the presence of at least one additive selected from the group formed by quaternary ammonium salts.

2. A process according to claim 1 characterised in that the catalyst solution results from the interaction of at least one chloro or bromo compound of aluminium hydrocarbyl with a mixture of at least one compound of zirconium and at least one organic compound selected from the class of acetals and cetals, resulting from the condensation of an aldehyde or a ketone with a monohydric alcohol or a polyhydric alcohol.

3. A process according to claim 1 characterised in that the concentration of zirconium in the catalytic solution is between 10⁻⁴ and 0.5 mole per litre.

4. A process according to one of claims 2 and 3 characterised in that the molar ratio between the aluminium compound and the zirconium compound is between 1:1 and 100:1.

5. A process according to one of claims 2 to 4 characterised in that the molar ratio between the acetal or the cetal and the zirconium compound is between 0.1:1 and 5:1.

6. A process according to one of the preceding claims characterised in that the zirconium compound is zirconium tetrachloride.

7. A process according to one of claims 2 to 6 characterised in that the cetal is di-(2-ethylhexyloxy)-2,2-propane.

8. A process according to one of the preceding claims characterised in that the aluminium compound is ethylaluminium sesquichloride.

9. A process according to one of the preceding claims characterised in that the quaternary ammonium salt is selected from the group formed by quaternary ammonium hydroxides or halides.

10. A process according to one of the preceding claims characterised in that the additive is selected from the group formed by quaternary ammonium chlorides.

11. A process according to one of the preceding claims characterised in that the quaternary ammonium comprises identical or different hydrocarbyl radicals, for example alkyl, cycloalkyl, aryl, cycloalkyl or aryl which are substituted by an alkyl group, comprising from 1 to 30 carbon atoms.

12. A process according to one of the preceding claims characterised in that the additive is used in the form of a solution in a hydrocarbon medium selected from the group formed by hydrocarbons, light alpha olefins and higher oligomers.

13. A process according to one of claims 1 to 11 characterised in that the additive is used in the pure state.

14. A process according to one of the preceding claims characterised in that, before proceeding with the conversion operation, the additive is introduced into the reaction enclosure to effect a treatment for passivation of the walls of the enclosure.

15. A process according to one of the preceding claims characterised in that the additive is introduced while the conversion reaction is taking place, the process being continuous.

16. A process according to one of claims 1 to 14 characterised in that, the process being discontinuous, the additive is introduced into the reaction enclosure with the catalyst solution.

17. A process according to one of the preceding claims characterised in that the additive is introduced independently of the catalyst solution.

18. A process according to one of the preceding claims characterised in that the amount of quaternary ammonium salt used during the conversion reaction is from 1 ppm to 5% by weight with respect to the oligomers produced.

19. A process according to one of the preceding claims characterised in that the amount of quaternary ammonium salt used during the conversion reaction is from 20 ppm to 5000 ppm with respect to the oligomers produced.

20. A process according to one of the preceding claims characterised in that the process takes place at a temperature of between 20 and 180°C under a pressure of from 0.5 to 15 MPa.
